(19) Europäisches Patentamt
European Patent Office
Office européen des brevets

(11) Veröffentlichungsnummer: **0 427 007 B1**

(12) # EUROPÄISCHE PATENTSCHRIFT

(45) Veröffentlichungstag der Patentschrift: **22.06.94**

(51) Int. Cl.5: **C09B 57/00**, G11B 7/24, C07C 233/10

(21) Anmeldenummer: **90119726.9**

(22) Anmeldetag: **15.10.90**

Die Akte enthält technische Angaben, die nach dem Eingang der Anmeldung eingereicht wurden und die nicht in dieser Patentschrift enthalten sind.

(54) **Amidgruppen aufweisende Azulenquadratsäurefarbstoffe, deren Zwischenprodukte sowie optisches Aufzeichnungsmedium.**

(30) Priorität: **25.10.89 DE 3935526**

(43) Veröffentlichungstag der Anmeldung:
**15.05.91 Patentblatt 91/20**

(45) Bekanntmachung des Hinweises auf die Patenterteilung:
**22.06.94 Patentblatt 94/25**

(84) Benannte Vertragsstaaten:
**CH DE FR GB IT LI**

(56) Entgegenhaltungen:
**EP-A- 0 310 080**
**EP-A- 0 341 541**

(73) Patentinhaber: **BASF Aktiengesellschaft**
**Carl-Bosch-Strasse 38**
**D-67063 Ludwigshafen(DE)**

(72) Erfinder: **Schmitt, Michael, Dr.**
**Freudenbergstrasse 18**
**W-6940 Weinheim(DE)**
Erfinder: **Albert, Bernhard, Dr.**
**Rietburgstrasse 13**
**W-6701 Maxdorf(DE)**
Erfinder: **Brosius, Sibylle, Dr.**
**Cordovastrasse 29**
**W-6700 Ludwigshafen(DE)**
Erfinder: **Schomann, Klaus Dieter, Dr.**
**Kopernikusstrasse 47**
**W-6700 Ludwigshafen(DE)**
Erfinder: **Kuppelmaier, Harald, Dr.**
**In den Bannzaeunen 17**
**W-6701 Goennheim(DE)**

**EP 0 427 007 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Amidgruppen aufweisende Azulenquadratsäurefarbstoffe der Formel I

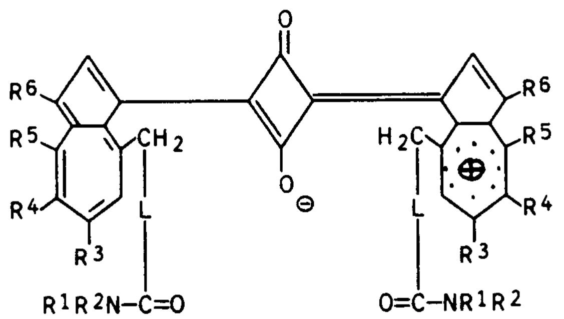

(I) ,

in der

| | |
|---|---|
| L | $C_1$-$C_{12}$-Alkylen, das gegebenenfalls durch Phenyl substituiert ist, |
| $R^1$ und $R^2$ | gleich oder verschieden sind und unabhängig voneinander jeweils Wasserstoff, gegebenenfalls substituiertes $C_1$-$C_{20}$-Alkyl, $C_5$-$C_7$-Cycloalkyl, gegebenenfalls substituiertes Phenyl, 2,2,6,6-Tetramethylpiperidin-4-yl oder Cyclohexylaminocarbonyl und |
| $R^3$, $R^4$, $R^5$ und $R^6$ | gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, |

bedeuten, mit der Maßgabe, daß wenn $R^6$ Wasserstoff bedeutet, an einem oder beiden Azulenringen die Ringpositionen der Substituenten $CH_2$-L-CO-$NR^1R^2$ und $R^5$ innerhalb eines Azulenringes auch gegeneinander vertauscht sein können, deren Zwischenprodukte sowie ein optisches Aufzeichnungsmedium, das die neuen Farbstoffe enthält.

Zur kostengünstigen Herstellung optischer Datenaufzeichnungsträger werden Farbstoffe mit besonderen Eigenschaften benötigt. Diese Farbstoffe sollten

- eine starke Absorption zwischen 700 und 900 nm aufweisen, um mit Halbleiterlasern beschreibbare Schichten zu liefern,
- in Schicht eine hohe Reflektivität im nahen Infrarot (700 bis 900 nm) aufweisen, um mit einem einfachen Schichtaufbau (ohne Reflektorschicht) auszukommen,
- eine hohe Löslichkeit aufweisen, um beispielsweise die dünne Speicherschicht durch Spincoating auf einen Träger aufbringen zu können und
- in dünnen Schichten eine hohe Stabilität aufweisen.

Die bisher bekannten Speichermaterialien weisen häufig zumindest in einem der genannten Anforderungspunkte Mängel auf.

Aufgabe der vorliegenden Erfindung war es daher, neue Farbstoffe bereitzustellen, bei denen die obengenannten Mängel nicht mehr oder höchstens nur in äußerst geringem Maße auftreten.

Demgemäß wurden die oben näher bezeichneten Amidgruppen aufweisende Azulenquadratsäurefarbstoffe der Formel I gefunden.

Alle in der obengenannten Formel I auftretenden Alkylen- und Alkylgruppen können sowohl geradkettig als auch verzweigt sein.

Wenn in Formel I substituierte Alkylgruppen auftreten, können als Substituenten z.B. $C_1$-$C_4$-Alkoxy, Phenyl, $C_1$-$C_4$-Alkylphenyl, $C_1$-$C_4$-Alkoxyphenyl, 2,2,6,6-Tetramethylpiperidin-4-ylaminocarbonyl oder 1-Acetyl-2,2,6,6-tetramethylpiperidin-4-ylaminocarbonyl in Betracht kommen.

Wenn in Formel I substituierte Phenylgruppen auftreten, können als Substituenten z.B. $C_1$-$C_4$-Alkyl, $C_1$-$C_4$-Alkoxy oder Halogen, dabei vorzugsweise Fluor, Chlor oder Brom, in Betracht kommen.

Reste L sind z.B. Methylen, Ethylen, 1,2- oder 1,3-Propylen, 1,2-, 1,3-, 2,3- oder 1,4-Butylen, Pentamethylen, Hexamethylen, Heptamethylen, Octamethylen, Nonamethylen, Decamethylen, Undecamethylen, Dodecamethylen, Phenylethylen, 1-Phenyl-1,3-propylen.

Reste $R^1$, $R^2$, $R^3$, $R^4$, $R^5$ und $R^6$ in Formel I sind beispielsweise Methyl, Ethyl, Propyl, Isopropyl, Butyl, Isobutyl, sec-Butyl, tert-Butyl, Pentyl, Isopentyl, Neopentyl, tert-Pentyl, Hexyl, 2-Methylpentyl, Heptyl, Octyl,

2-Ethylhexyl, Nonyl, Decyl, Undecyl oder Dodecyl.

Reste $R^1$ und $R^2$ sind weiterhin Tridecyl, Tetradecyl, Pentadecyl, Hexadecyl, Heptadecyl, Octadecyl, Nonadecyl, Eicosyl, Cyclopentyl, Cyclohexyl, Cycloheptyl, Phenyl, 2- oder 4-Methylphenyl, 2- oder 4-Methoxyphenyl, 2- oder 4-Chlorphenyl oder 2,4-Dichlorphenyl.

Reste $R^3$, $R^4$, $R^5$ und $R^6$ sind weiterhin z.B. Fluormethyl, Chlormethyl, Difluormethyl, Trifluormethyl, Trichlormethyl, 2-Fluorethyl, 2-Chlorethyl, 2-Bromethyl, 1,1,1-Trifluorethyl, Heptafluorpropyl, 4-Chlorbutyl, 5-Fluorpentyl, 6-Chlorhexyl, Cyanomethyl, 2-Cyanoethyl, 3-Cyanopropyl, 2-Cyanobutyl, 4-Cyanobutyl, 5-Cyanopentyl, 6-Cyanohexyl, 2-Methoxyethyl, 2-Ethoxyethyl, 2-Propoxyethyl, 2-Isopropoxyethyl, 2-Butoxyethyl, 2-Methoxypropyl, 2-Ethoxypropyl, 3-Ethoxypropyl, 2-Isopropoxypropyl, 4-Ethoxybutyl, 4-Isopropoxybutyl, 5-Ethoxypentyl, 6-Methoxyhexyl, Benzyl, 1-Phenylethyl, 2-Phenylethyl, 4-Chlorbenzyl, 4-Methoxybenzyl, 2-(4-Methylphenyl)ethyl, Methoxycarbonylmethyl, Ethoxycarbonylmethyl, 2-Methoxycarbonylethyl, 2-Ethoxycarbonylethyl, 3-Methoxycarbonylpropyl, 3-Ethoxycarbonylpropyl, 4-Methoxycarbonylbutyl, 4-Ethoxycarbonylbutyl, 5-Methoxycarbonylpentyl, 5-Ethoxycarbonylpentyl, 6-Methoxycarbonylhexyl oder 6-Ethoxycarbonylhexyl.

Bevorzugt sind Azulenquadratsäurefarbstoffe der Formel I, in der $R^3$, $R^4$, $R^5$ und $R^6$ jeweils $C_1$-$C_6$-Alkyl oder Wasserstoff bedeuten.

Besonders bevorzugt sind Azulenquadratsäurefarbstoffe der Formel I, in der $R^3$ und $R^5$ jeweils Methyl und $R^4$ und $R^6$ jeweils Wasserstoff bedeuten und L, $R^1$ und $R^2$ jeweils die obengenannte Bedeutung besitzen. Diese Farbstoffe entsprechen der Formel Ia

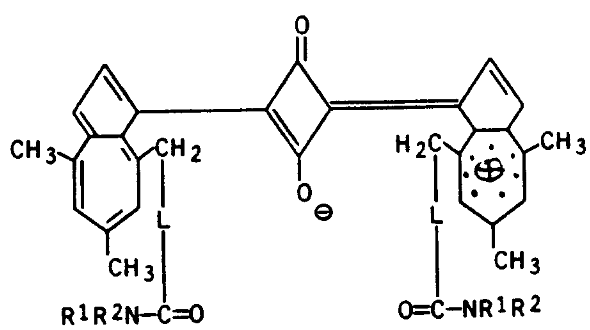

(Ia).

Ganz besonders bevorzugt sind Azulenquadratsäurefarbstoffe der Formel I, in der $R^3$ und $R^5$ jeweils Wasserstoff, $R^4$ Isopropyl und $R^6$ Methyl bedeuten und L, $R^1$ und $R^2$ jeweils die obengenannte Bedeutung besitzen. Diese Farbstoffe entsprechen der Formel Ib

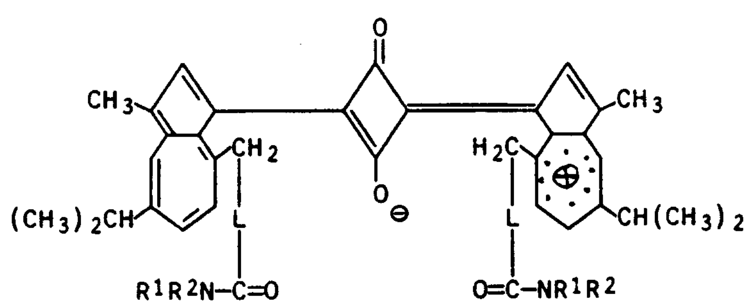

(Ib).

Weiterhin bevorzugt sind Azulenquadratsäurefarbstoffe der Formel I, in der $R^1$ und $R^2$ unabhängig voneinander Wasserstoff oder $C_1$-$C_{20}$-Alkyl, das gegebenenfalls durch Phenyl substituiert ist, bedeuten.

Die neuen Farbstoffe der Formel I werden aus Azulenderivaten der Formel II, in der L, $R^1$, $R^2$, $R^3$, $R^4$, $R^5$ und $R^6$ jeweils die obengenannte Bedeutung besitzen, durch Umsetzung mit Quadratsäure der Formel III gemäß der folgenden Gleichung erhalten:

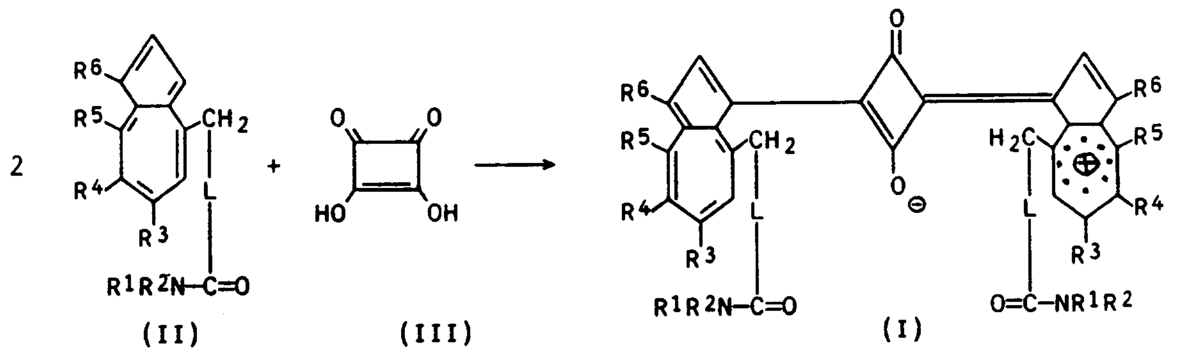

(II) (III) (I)

Bei denjenigen Azulenderivaten der Formel II, in denen $R^6$ Wasserstoff bedeutet, kann die Verknüpfung zur Quadratsäure an unterschiedlichen Ringpositionen des Fünfrings erfolgen, wobei isomere Produkte entstehen können, in denen die Ringpositionen der Substituenten $CH_2$-L-CO-$NR^1R^2$ und $R^5$, wie oben ausgeführt, gegeneinander vertauscht sind. Es sind nämlich dann Verbindungen, bei denen die Bindung zur Quadratsäure an derjenigen Seite erfolgt, an der der Substituent $CH_2$-L-CO-$NR^1R^2$ gebunden ist, von solchen zu unterscheiden, bei denen die Bindung zur Quadratsäure an derjenigen Seite erfolgt, an der der Substituent $R^6$ gebunden ist. Diese isomeren Verbindungen können chromatographisch getrennt werden. Für die Anwendung in Speicherschichten werden jedoch üblicherweise die Isomerengemische eingesetzt.

Das Herstellverfahren ist an sich bekannt und beispielsweise in Angew. Chem. 78, 937 (1966), oder in der EP-A-310 080 beschrieben.

Die Erfindung betrifft weiterhin neue Amidgruppen aufweisende Azulene der Formel II

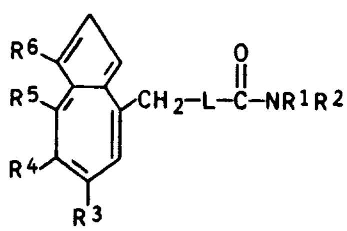

(II),

in der

| | |
|---|---|
| L | $C_1$-$C_{12}$-Alkylen, das gegebenenfalls durch Phenyl substituiert ist, |
| $R^1$ und $R^2$ | gleich oder verschieden sind und unabhängig voneinander jeweils Wasserstoff, gegebenenfalls substituiertes $C_1$-$C_{20}$-Alkyl, $C_5$-$C_7$-Cycloalkyl, gegebenenfalls substituiertes Phenyl, 2,2,6,6-Tetramethylpiperidin-4-yl oder Cyclohexylaminocarbonyl und |
| $R^3$, $R^4$, $R^5$ und $R^6$ | gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, |

bedeuten.

Bezüglich der beispielhaften Veranschaulichung der Reste L, $R^1$, $R^2$, $R^3$, $R^4$, $R^5$ und $R^6$ sei auf die voranstehend vorgenommene Aufzählung verwiesen.

Die Amidgruppen aufweisenden Azulenderivate der Formel II erhält man beispielsweise, wenn man von den entsprechenden freien Azulenalkylcarbonsäuren der Formel IV

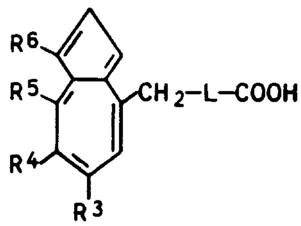

(IV),

in der $R^3$, $R^4$, $R^5$ und $R^6$ jeweils die obengenannte Bedeutung besitzen, ausgeht. Die Herstellung dieser Produkte ist beispielsweise in der EP-A-310 080 beschrieben.

So eignen sich z.B. diejenigen Azulenderivate der Formel IVa oder IVb

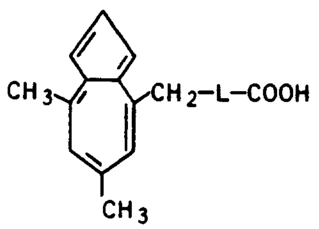

(IVa)

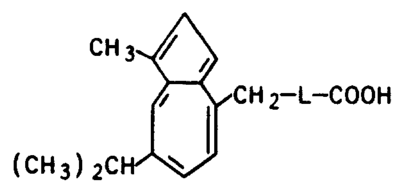

(IVb)

wobei L jeweils die obengenannte Bedeutung besitzt, besonders gut zur Umsetzung.

Als Reaktionspartner für die Azulenalkylcarbonsäuren IV dienen organische primäre oder sekundäre Amine der Formel V

$R^1R^2NH$ (V),

in der $R^1$ und $R^2$ jeweils die obengenannte Bedeutung besitzen.

Die Umsetzung der Azulenalkylcarbonsäuren IV mit den Aminen V erfolgt nach an sich bekannten Methoden. Dazu kann z.B. die Azulenalkylcarbonsäure mit dem Amin in einem inerten organischen Lösungsmittel (z.B. Dichlormethan, 1,1,2-Trichlorethan, Toluol, Ligroin oder Cyclohexan) mit einem Kondensationsmittel (z.B. Dicyclohexylcarbodiimid) bei einer Temperatur von 10 bis 60 °C, gegebenenfalls in Gegenwart eines Katalysators (z.B. tertiäre Amine) umgesetzt werden. Im allgemeinen wird dabei das Amin V im Überschuß verwendet.

Eine weitere Aufgabe der vorliegenden Erfindung war es, ein neues optisches Aufzeichnungsmedium mit Azulenquadratsäurederivaten als Speichermaterialien bereitzustellen, das in einfacher Weise hergestellt werden kann, das gut beschreibbar und anschließend auch gut lesbar ist, wobei das Signal:Rausch-Verhältnis möglichst hoch sein sollte, und das eine hohe Stabilität der Speicherschichten aufweist.

Die Erfindung betrifft daher weiterhin ein optisches Aufzeichnungsmedium, enthaltend einen Träger sowie einen strahlungsempfindlichen, Farbstoff und gegebenenfalls Bindemittel enthaltenden dünnen Beschichtungsfilm, wobei der Farbstoff die Formel I

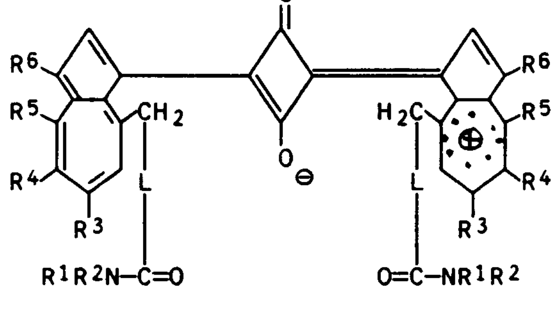

(I)

aufweist, in der

| | |
|---|---|
| L | $C_1$-$C_{12}$-Alkylen, das gegebenenfalls durch Phenyl substituiert ist, |
| $R^1$ und $R^2$ | gleich oder verschieden sind und unabhängig voneinander jeweils Wasserstoff, gegebenenfalls substituiertes $C_1$-$C_{20}$-Alkyl, $C_5$-$C_7$-Cycloalkyl, gegebenenfalls substituiertes Phenyl, 2,2,6,6-Tetramethylpiperidin-4-yl oder Cyclohexylaminocarbonyl und |
| $R^3$, $R^4$, $R^5$ und $R^6$ | gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, |

bedeuten, mit der Maßgabe, daß wenn $R^6$ Wasserstoff bedeutet, an einem oder beiden Azulenringen die Ringpositionen der Substituenten $CH_2$-L-CO-$NR^1R^2$ und $R^5$ innerhalb eines Azulenringes auch gegeneinander vertauscht sein können.

Bevorzugt ist ein optisches Aufzeichnungsmedium, das Azulenquadratsäurefarbstoffe der Formel I enthält, in der $R^3$, $R^4$, $R^5$ und $R^6$ jeweils $C_1$-$C_6$-Alkyl oder Wasserstoff bedeuten.

Besonders bevorzugt ist ein optisches Aufzeichnungsmedium, das Azulenquadratsäurefarbstoffe der Formel I enthält, in der $R^3$ und $R^5$ jeweils Methyl und $R^4$ und $R^6$ jeweils Wasserstoff bedeuten.

Ganz besonders bevorzugt ist ein optisches Aufzeichnungsmedium, das Azulenquadratsäurefarbstoffe der Formel I enthält, in der $R^3$ und $R^5$ jeweils Wasserstoff, $R^4$ Isopropyl und $R^6$ Methyl bedeuten.

Weiterhin bevorzugt ist ein optisches Aufzeichnungsmedium, das Azulenquadratsäurefarbstoffe der Formel I enthält, in der $R^1$ und $R^2$ unabhängig voneinander Wasserstoff oder $C_1$-$C_{20}$-Alkyl, das gegebenenfalls durch Phenyl substituiert ist, bedeuten.

Als Träger kommen zweckmäßig transparente Träger, wie Glas oder Kunststoffe in Betracht. Geeignete Kunststoffe sind beispielsweise Poly(meth)acrylate, Polycarbonate, Polyester, Epoxide, Polyolefine (z.B. Polymethylpenten), Polyamid, Polyvinylchlorid, Polystyrol oder Polyvinylester.

Ein bevorzugtes Aufzeichnungsmedium weist einen Träger aus Polycarbonat oder Poly(meth)acrylat, insbesondere aber Polycarbonat auf.

Weiterhin bevorzugt ist ein optisches Aufzeichnungsmedium, das 1 bis 30 Gew.-%, bezogen auf Farbstoff, an Bindemittel enthält.

Die neuen Azulenquadratsäurefarbstoffe der Formel I zeigen gute optische Daten. Darüber hinaus sind bei den neuen Verbindungen die reinen Farbstoffschichten sehr stabil. Es wurde nämlich keine Rekristallisation der reinen Farbstoffschicht beobachtet und somit kann auf den Zusatz polymerer Bindemittel auch verzichtet werden. Außerdem ist auch die Lichtechtheit (Stabilität) deutlich größer als bei bekannten Methinfarbstoffe, so daß der Zusatz von Stabilisatoren zur Schichtrezeptur auf ein Minimum begrenzt werden kann. Von besonderem Vorteil ist auch die gute Löslichkeit der neuen Farbstoffe I in den meisten organischen Lösungsmitteln, so daß diese Farbstoffe direkt (ohne Schutzschicht) auf strukturierte Kunststoffsubstrate, insbesondere Polycarbonatsubstrate, aufgeschleudert werden können.

Wie oben ausgeführt, enthält die aufzuschleudernde Lösung vorzugsweise ein Bindemittel, um eine gute Langzeitstabilität des Aufzeichnungsmediums zu gewährleisten und vor allem die optimale Viskosität der aufzuschleudernden Lösung einzustellen. Vorzugsweise enthält die Lösung dabei 1 bis 30 Gew.-%, bezogen auf den Gehalt an gelöstem Feststoff der aufzuschleudernden Lösung, eines Bindemittels.

Als Bindemittel kommen z.B. Polyorganosiloxane, Epoxide, Poly(meth)acrylate, Polystyrolhomo- und copolymerisate, Polyvinylcarbazol, Polyvinylpyrrolidon, Polyimidazolcopolymere, Polyvinylestercopolymere, Polyvinylethercopolymere, Polyvinylidenchloridpolymere, Acrylnitrilcopolymere, Polyvinylchlorid oder dessen Copolymere, Celluloseacetat oder Nitrocellulose in Betracht.

Ein bevorzugtes Aufzeichnungsmedium weist ein Bindemittel auf Basis eines Vinylpyrrolidon-Vinylacetat-Copolymeren oder eines Polyvinylchlorid-Polyvinylether-Copolymeren auf.

Das erfindungsgemäße optische Aufzeichnungsmedium wird zweckmäßig durch Aufschleudern einer Lösung, enthaltend organisches Lösungsmittel, Azulenquadratsäurefarbstoff I und gegebenenfalls Bindemittel hergestellt. Zweckmäßig weist die aufzuschleudernde Lösung dabei einen Gehalt an gelösten Feststoffen von 1 bis 30 Gew.-%, bezogen auf die Lösung, auf.

Geeignete Lösungsmittel sind z.B. Propanol, Isopropanol, Butanol, Diacetonalkohol, Methylethylketon, Toluol, Bromoform, 1,1,2-Trichlorethan oder deren Mischungen.

Gegebenenfalls kann die Lösung noch bis zu 10 Gew.-%, bezogen auf den Gehalt an gelösten Feststoffen der aufzuschleudernden Lösung, an Additiven, z.B. Antioxidantien, Singulett-Sauerstoff-Quencher oder UV-Absorber enthalten.

Bevorzugt enthält die aufzuschleudernde Lösung bis zu 5 Gew.-%, bezogen auf den Gehalt an gelösten Feststoffen der aufzuschleudernden Lösung, eine Mischung von mehreren Antioxidantien, Singulett-Sauerstoff-Quenchern und UV-Absorbern. Bei Anwendung von solchen Antioxidantien, die ebenfalls im nahen

Infrarot absorbieren, beispielsweise Nickelthiolenkomplexe, wie sie z.B. in der DE-A-3 505 750, DE-A-3 505 751 oder in Dyes and Pigments, Bd. 8, S. 381-388 (1987), beschrieben sind, können vorzugsweise bis zu 10 Gew.-%, bezogen auf den Gehalt an gelösten Feststoffen der aufzuschleudernden Lösung, in der Lösung enthalten sein.

Unter Aufschleudern ist dabei das Aufbringen der Lösung auf den in Rotation befindlichen Träger, der zweckmäßig eine runde Form aufweist, zu verstehen. Es ist aber auch möglich, die Lösung auf den zunächst ruhenden Träger aufzubringen und ihn anschließend in Rotation zu versetzen. Das Aufgeben auf den Träger erfolgt zweckmäßig mit einer Spritze oder Kapillaren oder mittels einer mechanischen Pumpe.

Die Rotation des Trägers erfolgt im allgemeinen mit einer Geschwindigkeit von 5 bis 7000 U/min, vorzugsweise 500 bis 5000 U/min, wobei das Aufschleudern der Lösung zweckmäßig bei geringerer Drehzahl (ca. 500 bis 2000 U/min) und das daran anschließende Trockenschleudern bei höherer Drehzahl (ca. 5000 bis 7000 U/min) vorgenommen wird. Die Schichtdicke der gegenüber Laserlicht empfindlichen Schicht beträgt 40 bis 160 nm, vorzugsweise 80 bis 120 nm. Sie ist abhängig von der Drehzahl, von der Konzentration und Viskosität der aufzuschleudernden Lösung sowie der Temperatur.

Beim erfindungsgemäßen optischen Aufzeichnungsmedium liegt die gegenüber Laserlicht empfindliche Schicht in Form einer homogenen, dünnen, glatten Schicht vor, die eine hohe optische Qualität aufweist. So liegen die Reflektivitätswerte im allgemeinen in einem Bereich der größer als 12 % ist.

Das neue Aufzeichnungsmedium ist ferner bei der Wellenlänge der verwendeten Laserlichtquelle hinreichend empfindlich, d.h. bei Einstrahlung von Lichtpulsen von wenigen nJ Energiegehalt, die auf Brennpunktdurchmesser von $\leqq 1\ \mu m$ fokussiert sind, bilden sich Pits aus, wobei ein ausgezeichnetes Signal:Rausch-Verhalten erzielt wird.

Als Laserlichtquelle eignen sich wegen der geringen Größe des Bauelementes, seines geringen Energiebedarfs und der Möglichkeit der direkten Modulation der optischen Ausgangsleistung durch Modulation des elektrischen Antriebsstromes Festkörper-Injektionslaser, die im nahen Infrarot emittieren, vor allem der AlGaAs-Laser, der im Wellenlängenbereich zwischen etwa 750 und 900 nm arbeitet, besonders gut.

Die folgenden Beispiele sollen die Erfindung näher erläutern.

Beispiel 1

Herstellung von N-Butyl-3-(7-isopropyl-1-methylazulen-4-yl)propionsäureamid

In eine Lösung von 5,1 g (0,02 mol) (7-Isopropyl-1-methylazulen-4-yl)-propionsäure in 50 ml Dichlormethan wurden 4,9 g (0,024 mol) Dicyclohexylcarbodiimid in 10 ml Dichlormethan bei 0°C getropft und 30 Minuten gerührt. Danach wurden 3,7 g (0,05 mol) n-Butylamin zugegeben und 2 Stunden bei Raumtemperatur gerührt. Vom Niederschlag (Dicyclohexylharnstoff) wurde abfiltriert. Das Filtrat wurde mehrmals mit 2N Salzsäure gewaschen, über Natriumsulfat getrocknet, danach wurde das Lösungsmittel abgezogen. Der Rückstand wurde an Kieselgel chromatographiert (Ethylacetat:Petrolether 9:1 v/v). Man erhielt 2,9 g (47 %) des Zielproduktes als blaues hochviskoses öl.

Physikalische Daten:

IR (KBr): 3304 (N-H); 2954, 2931, 2852, (C-H); 1643, 1627 (C=O); 1556, 1464, 1435, 1387 $cm^{-1}$.-$^1$H-NMR ($CDCl_3$):$\delta$ = 0.95 (t, 3H); 1.22 (m, 6H); 1.35 (d, 6H); 2.65 (s, 3H); 3.05 (m, 1H); 3.15 (m, 2H); 3.48 (t, 2H); 5.50 (bs, 1H); 7.00 (d, 1H); 7.29 (2, 1H); 7.38 (d, 1H); 7,60 (d, 1H); 8.18 (s, 1H)ppm.-$^{13}$C-NMR ($CDCl_3$):$\delta$ = 12.83; 13.65; 20.02; 24.69 (2C); 31.68; 34.00; 38.00; 38.27; 39.35; 112.26; 124.50; 125.46; 133.29; 135.18; 136.49; 136.69; 136.99; 140.24; 147.21; 172,03 ppm.-MS: e/m = 311 (35 %, $M^+$).-

Analog Beispiel 1 wurden die in Tabelle 1 aufgeführten Azulenderivate hergestellt.

Alle Strukturen sind durch IR-, $^{1}H$-NMR-, $^{13}C$-NMR- und MS-Spektren abgesichert.

Tabelle 1

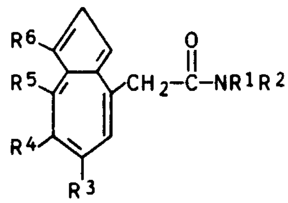

| Bei-spiel-Nr. | $L-C(O)NR^1R^2$ | $R^3$ | $R^4$ | $R^5$ | $R^6$ | MS[M⊕] | IR($\tilde{\nu}$/$cm^{-1}$) [NH; C=O] |
|---|---|---|---|---|---|---|---|
| 2 | 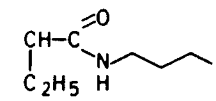 | H | $CH(CH_3)_2$ | H | $CH_3$ | 339.5 | 3294; 1645 |
| 3 | 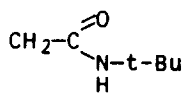 | H | $CH(CH_3)_2$ | H | $CH_3$ | 311.5 | 3280; 1640 |
| 4 | 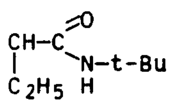 | H | $CH(CH_3)_2$ | H | $CH_3$ | 339.5 | 3312; 1639 |
| 5 | 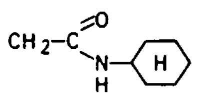 | H | $CH(CH_3)_2$ | H | $CH_3$ | 337.5 | 3301; 1642 |
| 6 | 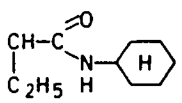 | H | $CH(CH_3)_2$ | H | $CH_3$ | 365.5 | 3312; 1636 |
| 7 | 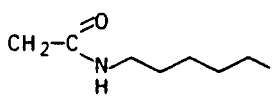 | H | $CH(CH_3)_2$ | H | $CH_3$ | 339.5 | 3291; 1643 |
| 8 | 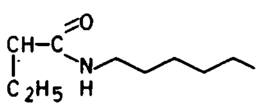 | H | $CH(CH_3)_2$ | H | $CH_3$ | 367.5 | 3294; 1644 |
| 9 | 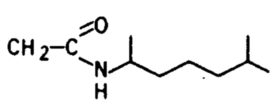 | H | $CH(CH_3)_2$ | H | $CH_3$ | 367.5 | 3289; 1640 |
| 10 | 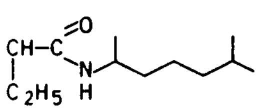 | H | $CH(CH_3)_2$ | H | $CH_3$ | 395.6 | 3291; 1637 |
| 11 | 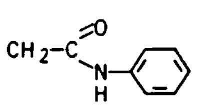 | H | $CH(CH_3)_2$ | H | $CH_3$ | 331.5 | 3313; 1663 |
| 12 | 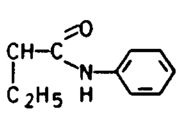 | H | $CH(CH_3)_2$ | H | $CH_3$ | 359.5 | 3300; 1657 |

Fortsetzung - Tabelle 1

| Bei-spiel-Nr. | L-C(O)NR¹R² | $R^3$ | $R^4$ | $R^5$ | $R^6$ | MS[M⊕] | IR($\tilde{\nu}$/cm⁻¹) [NH; C=O] |
|---|---|---|---|---|---|---|---|
| 13 | 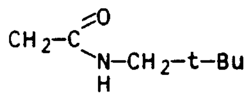 | H | $CH(CH_3)_2$ | H | $CH_3$ | 325.5 | 3321; 1637 |
| 14 | 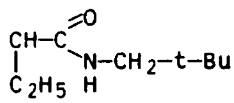 | H | $CH(CH_3)_2$ | H | $CH_3$ | 353.5 | 3306; 1657 |
| 15 | 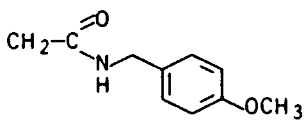 | H | $CH(CH_3)_2$ | H | $CH_3$ | 375.52 | 3301; 1639 |
| 16 | 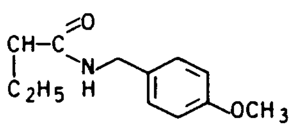 | H | $CH(CH_3)_2$ | H | $CH_3$ | 403.5 | 3301; 1639 |
| 17 | 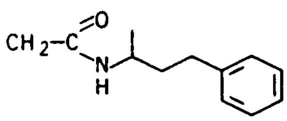 | H | $CH(CH_3)_2$ | H | $CH_3$ | 387.5 | 3319; 1638 |
| 18 | 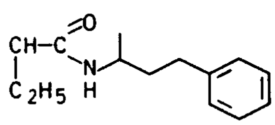 | H | $CH(CH_3)_2$ | H | $CH_3$ | 415.5 | 3309; 1638 |
| 19 | 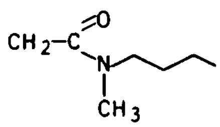 | H | $CH(CH_3)_2$ | H | $CH_3$ | 325.5 | — ; 1646 |
| 20 | 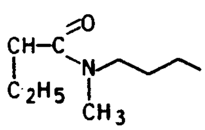 | H | $CH(CH_3)_2$ | H | $CH_3$ | 353.5 | — ; 1640 |
| 21 | 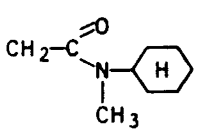 | H | $CH(CH_3)_2$ | H | $CH_3$ | 351.5 | — ; 1641 |
| 22 | 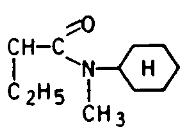 | H | $CH(CH_3)_2$ | H | $CH_3$ | 379.5 | — ; 1634 |
| 23 | 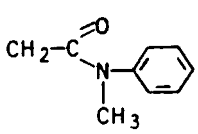 | H | $CH(CH_3)_2$ | H | $CH_3$ | 345.5 | — ; 1658 |

Fortsetzung - Tabelle 1

| Bei-spiel-Nr. | L-C(O)NR1R2 | R3 | R4 | R5 | R6 | MS [M⊕] | IR ($\tilde{\nu}$/cm-1) [NH; C=O] |
|---|---|---|---|---|---|---|---|
| 24 | 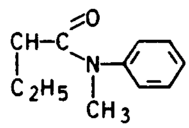 | H | $CH(CH_3)_2$ | H | $CH_3$ | 373.5 | — ; 1654 |
| 25 | 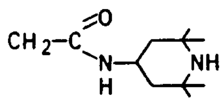 | H | $CH(CH_3)_2$ | H | $CH_3$ | 394.61 | 3284; 1637 |
| 26 | 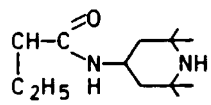 | H | $CH(CH_3)_2$ | H | $CH_3$ | 422.6 | 3275; 1639 |
| 27 | 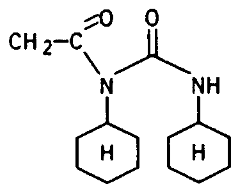 | H | $CH(CH_3)_2$ | H | $CH_3$ | 462.6 | 3271; 1704; 1639 |
| 28 | 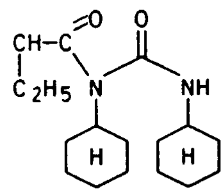 | H | $CH(CH_3)_2$ | H | $CH_3$ | 490.7 | 3350; 3405; 1705; 1657 |
| 29 | 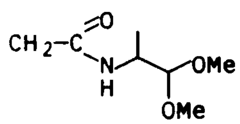 | H | $CH(CH_3)_2$ | H | $CH_3$ | 357.5 | 3309; 1642 |
| 30 | 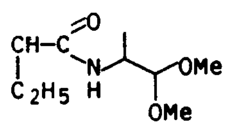 | H | $CH(CH_3)_2$ | H | $CH_3$ | 385.5 | 3305; 1639 |
| 31 | 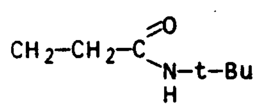 | H | $CH(CH_3)_2$ | H | $CH_3$ | 325.5 | 3290; 1637 |

Beispiel 32

Herstellung von (7-Isopropyl-1-methylazulen-4-yl)propionsäureamid

Zu einer Lösung von 23,6 g (0,12 Mol) Guajazulen und 26,4 ml (0,19 mol) Diisopropylamin in 400 ml Methyl-t-butylether wurden bei -10 °C innerhalb 1 Stunde 100 ml Butyllithium-Lösung (in Hexan, 0,16 mol) langsam zugetropft und 1 Stunde bei 0 °C gerührt. Danach wurde eine Suspension von 14,0 g (0,15 mol) Chloracetamid zugegeben und 12 Stunden bei Raumtemperatur gerührt. Das Reaktionsgemisch wurde mit 200 ml Wasser hydrolysiert. Die organische Phase wurde mehrmals mit Wasser gewaschen und über Natriumsulfat getrocknet. Nach Abziehen des Lösungsmittels unter vermindertem Druck wurde der Rückstand an Kieselgel chromatographiert. Man erhielt 7,1 g (23 %) des Amids als blaue Kristalle vom Schmp.

149-152 °C.

Physikalische Daten:

IR (KBr): 3186 (N-H); 2961, (C-H); 1651 (C=O); 1456, 1433, 1403, 774 $cm^{-1}$.-$^{1}H$-NMR ($CDCl_3$):$\delta$=1.35 (d, 6H); 2.65 (s, 3H); 2.70 (m, 2H); 3.08 (m, 1H); 3.30 (t, 2H); 5.50 (bs, 2H); 7.00 (d, 1H); 7.28 (d, 1H); 7.38 (d, 1H); 7,60 (d, 1H); 8.15 (s, 1H)ppm.-$^{13}C$-NMR ($CDCl_3$):$\delta$=12.82; 24.69; (2C); 33.62; 37.05; 38.28; 112.25; 124.37; 125.60; 133.37; 135.26; 136.61; 136.78; 136.99; 140.38; 146.93; 174.58 ppm.-MS: e/m = 255 (100 %, $M^+$).-

Beispiel 33

Darstellung des Bis[N-butyl-(7-isopropyl-1-methylazulen-4-yl)propionsäureamid]-quadratsäurefarbstoffs

Eine Mischung von 2,7 g (4 mmol) N-(n-Butyl)-3-(7-isopropyl-1-methylazulen-4-yl)propionsäureamid, 1,0 g (8,7 mmol) Quadratsäure, 40 ml n-Butanol und 40 ml Toluol wurde am Wasserabscheider 3 Stunden unter Rückfluß erhitzt. Nach Abdestillieren des Lösungsmittels wurde der Rückstand aus Ethylacetat/Methanol umkristallisiert. Man erhielt 1,4 g (50 %) Farbstoff als metallisch glänzende Kristalle vom Schmp. 122-125 °C.

Physikalische Daten:

UV ($CH_2Cl_2$): $\lambda_{max}(\epsilon)$ = 775 (119 000) nm.- IR (KBr): 3300 (N-H); 2958, (C-H); 1643, 1643, 1612 (C=O); 1546, 1435, 1387, 1332, 1251, 1072, 1013 $cm^{-1}$.-$^{1}H$-NMR ($CDCl_3$):$\delta$=0.85 (t, 6H); 1.05-1.45 (m, 8H); 1.35 (d, 12H); 2.55 (s, 6H); 2.73 (t, 4H); 3.10 (cm, 6H); 4.18 (t, 4H); 6.68 (bs, 2H); 7.65 (s, 4H); 8.10 (s, 2H); 8.95 (s, 2H) ppm.-$^{13}C$-NMR ($CDCl_3$):$\delta$=13.04 (2C); 13.64 (2C); 20.08 (2C); 24.19 (4C); 31.61 (2C); 35.55 (2C); 37.85 (2C); 38.35 (2C); 39.34 (2C); 121.22 (2C); 131.22 (2C); 134.26 (2C); 134.72 (2C); 138.49 (2C); 140.24 (2C); 141.80 12C); 148.17 (2C); 151.09 (2C); 154.60 (2C); 171.99 (2C); 180.57 (2C); 183.68 (2C); ppm.- MS: e/m = 700 (15 %, $M^+$).-

Analog Beispiel 33 wurden die in Tabelle 2 aufgeführten Quadratsäurefarbstoffe hergestellt und zusätzlich durch IR-, $^{1}H$-NMR-, $^{13}C$-NMR- und MS-Spektren charakterisiert.

Tabelle 2

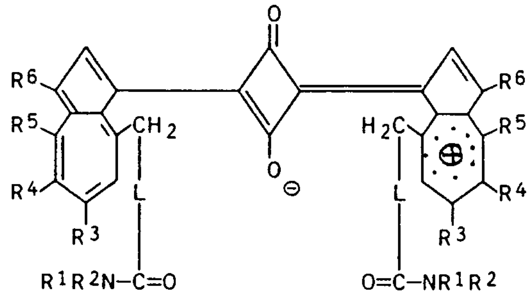

| Beispiel-Nr. | L-C(O)NR[1]R[2] | $R^3$ | $R^4$ | $R^5$ | $R^6$ | λmax (ε) [nm] | Schmp. [°C] |
|---|---|---|---|---|---|---|---|
| 34 | 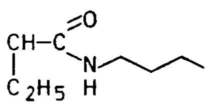 | H | $CH(CH_3)_2$ | H | $CH_3$ | 774 (107 000) | 147 - 150 |
| 35 | 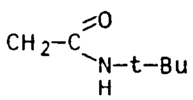 | H | $CH(CH_3)_2$ | H | $CH_3$ | 776 (116 000) | 213 - 214 |
| 36 | 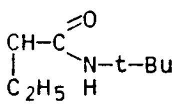 | H | $CH(CH_3)_2$ | H | $CH_3$ | 775 (107 000) | 240 - 241 |
| 37 | 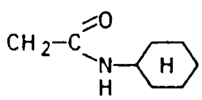 | H | $CH(CH_3)_2$ | H | $CH_3$ | 778 (116 000) | 242 - 245 |
| 38 | 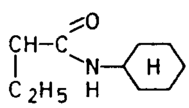 | H | $CH(CH_3)_2$ | H | $CH_3$ | 774 (104 000) | 218 - 222 |
| 39 | 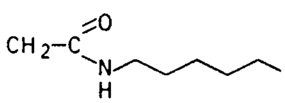 | H | $CH(CH_3)_2$ | H | $CH_3$ | 775 (116 000) | 234 - 237 |
| 40 | 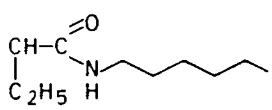 | H | $CH(CH_3)_2$ | H | $CH_3$ | 774 (111 000) | 232 - 235 |
| 41 | 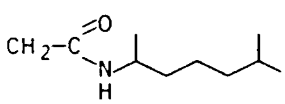 | H | $CH(CH_3)_2$ | H | $CH_3$ | 770 (125 000) | 205 - 207 |
| 42 | 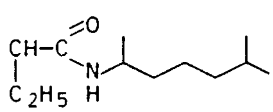 | H | $CH(CH_3)_2$ | H | $CH_3$ | 768 (112 000) | 176 - 180 |

Fortsetzung - Tabelle 2

| Beispiel-Nr. | L-C(O)NR¹R² | R³ | R⁴ | R⁵ | R⁶ | λmax (ε) [nm] | Schmp. [°C] |
|---|---|---|---|---|---|---|---|
| 43 | 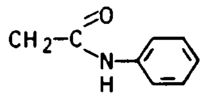 | H | $CH(CH_3)_2$ | H | $CH_3$ | 772 (141 000) | 263 - 264 |
| 44 | 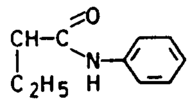 | H | $CH(CH_3)_2$ | H | $CH_3$ | 770 (127 000) | 247 - 248 |
| 45 | 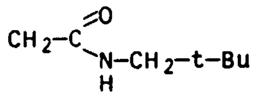 | H | $CH(CH_3)_2$ | H | $CH_3$ | 771 (137 000) | 214 - 216 |
| 46 | 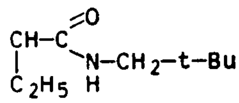 | H | $CH(CH_3)_2$ | H | $CH_3$ | 769 (121 000) | 229 - 230 |
| 47 | 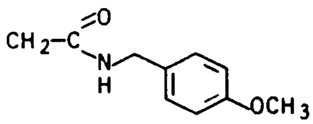 | H | $CH(CH_3)_2$ | H | $CH_3$ | 770 (120 000) | 223 - 226 |
| 48 | 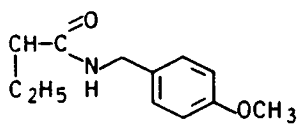 | H | $CH(CH_3)_2$ | H | $CH_3$ | 772 (113 000) | 224 - 226 |
| 49 | 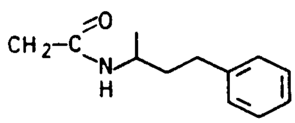 | H | $CH(CH_3)_2$ | H | $CH_3$ | 771 (124 000) | 205 - 207 |
| 50 | 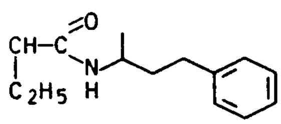 | H | $CH(CH_3)_2$ | H | $CH_3$ | 769 (111 000) | 224 - 226 |
| 51 | 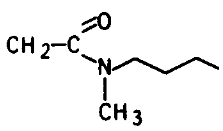 | H | $CH(CH_3)_2$ | H | $CH_3$ | 771 (114 000) | 132 - 134 |
| 52 | 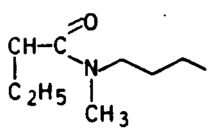 | H | $CH(CH_3)_2$ | H | $CH_3$ | 771 (109 000) | 205 - 207 |
| 53 | 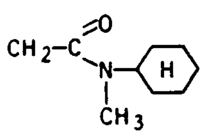 | H | $CH(CH_3)_2$ | H | $CH_3$ | 769 (118 000) | 186 - 188 |

Fortsetzung - Tabelle 2

| Beispiel-Nr. | $L-C(O)NR^1R^2$ | $R^3$ | $R^4$ | $R^5$ | $R^6$ | λmax (ε) [nm] | Schmp. [°C] |
|---|---|---|---|---|---|---|---|
| 54 | 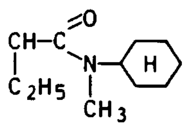 | H | $CH(CH_3)_2$ | H | $CH_3$ | 771 (120 000) | 152 - 154 |
| 55 | 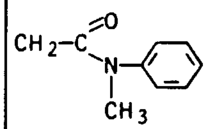 | H | $CH(CH_3)_2$ | H | $CH_3$ | 768 (116 000) | 236(Zers.) |
| 56 | 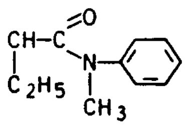 | H | $CH(CH_3)_2$ | H | $CH_3$ | 770 (117 000) | 210(Zers.) |

Beispiel 57

Eine 5 gew.-%ige Lösung des Farbstoffs aus Beispiel 33 in Toluol wurde mit einer Spritze bei ca. 2000 U/min auf eine rotierende Polymethylmethacrylatscheibe aufgetragen und dann das restliche Lösungsmittel bei 5000 U/min abgeschleudert. Man erhielt eine homogene, hochreflektierende Farbstoffschicht, welche sich mit einem Halbleiterlaser (λ = 830 nm) sehr gut beschreiben ließ. Die Informationen können mit sehr gutem Kontrast wieder ausgelesen werden.

Beispiel 58

Eine 3 gew.-%ige Lösung des Farbstoffs aus Beispiel 33 in Propanol/Diacetonalkohol (1:1 v/v), welche 30 Gew.-%, bezogen auf den Gehalt an gelöstem Feststoff der Lösung, Polymethylmethacrylat enthielt, wurde analog Beispiel 57 auf eine gerillte Polycarbonatscheibe aufgeschleudert. Man erhielt eine homogene, hochreflektierende Farbstoffschicht, die auf dem Substrat gut haftet, die Spurrillen des Substrates gut abbildet und mit einem Halbleiterlaser (λ = 830 nm) sehr gut beschreibbar ist. Die eingeschriebene Informationen war im Klimatest stabil und kann mit gutem Kontrast beliebig oft wieder ausgelesen werden.

Beispiel 59

Eine 2 gew.-%ige Lösung des Farbstoffs aus Beispiel 33 in Propanol/Diacetonalkohol (1:1 v/v), welche, bezogen auf den Gehalt an gelöstem Feststoff der Lösung, 10 Gew.-% eines Phenolharzes als Bindemittel und 5 Gew.-% 4-Octyl-4'-fluor-diphenyl-dithiolennickel als Stabilisator enthielt, wurde analog Beispiel 57 auf eine gerillte Polycarbonatscheibe aufgeschleudert. Die erhaltene Speicherschicht war derjenigen aus Beispiel 57 in allen Belangen vergleichbar, wies aber eine erhöhte Stabilität gegenüber UV-Licht auf.

Beispiel 60

Eine 2 gew.-%ige Lösung des Farbstoffs aus Beispiel 33 in Toluol, welche, bezogen auf den Gehalt an gelöstem Feststoff der Lösung, 10 Gew.-% Polymethylmethacrylat und 5 Gew.-% Biscampheratodithiolennickel enthielt, wurde analog Beispiel 57 auf eine Glasscheibe aufgeschleudert. Die erhaltene Farbstoffschicht war homogen und zeigte eine hohe Grundreflektivität. Sie konnte mit einem Halbleiterlaser (λ = 780 nm) gut beschrieben werden. Die eingeschriebenen Informationen sind unter den üblichen Testbedingungen stabil und können beliebig oft wieder ausgelesen werden.

## Patentansprüche

1. Amidgruppen aufweisende Azulenquadratsäurefarbstoffe der Formel I

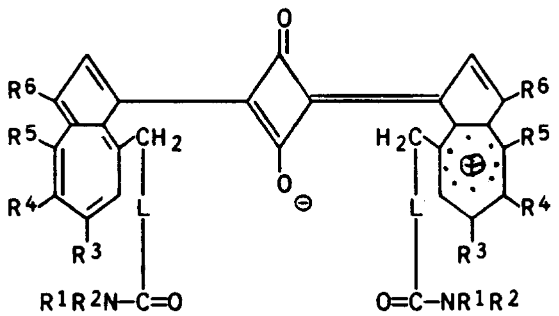

(I),

in der

| | |
|---|---|
| L | $C_1$-$C_{12}$-Alkylen, das gegebenenfalls durch Phenyl substituiert ist, |
| $R^1$ und $R^2$ | gleich oder verschieden sind und unabhängig voneinander jeweils Wasserstoff, gegebenenfalls substituiertes $C_1$-$C_{20}$-Alkyl, $C_5$-$C_7$-Cycloalkyl, gegebenenfalls substituiertes Phenyl, 2,2,6,6-Tetramethylpiperidin-4-yl oder Cyclohexylaminocarbonyl und |
| $R^3$, $R^4$, $R^5$ und $R^6$ | gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, |

bedeuten, mit der Maßgabe, daß wenn $R^6$ Wasserstoff bedeutet, an einem oder beiden Azulenringen die Ringpositionen der Substituenten $CH_2$-L-CO-$NR^1R^2$ und $R^5$ innerhalb eines Azulenringes auch gegeneinander vertauscht sein können.

2. Azulenquadratsäurefarbstoffe nach Anspruch 1, dadurch gekennzeichnet, daß $R^3$, $R^4$, $R^5$ und $R^6$ jeweils $C_1$-$C_6$-Alkyl oder Wasserstoff bedeuten.

3. Azulenquadratsäurefarbstoffe nach Anspruch 1, dadurch gekennzeichnet, daß $R^3$ und $R^5$ jeweils Methyl und $R^4$ und $R^6$ jeweils Wasserstoff bedeuten.

4. Azulenquadratsäurefarbstoffe nach Anspruch 1, dadurch gekennzeichnet, daß $R^3$ und $R^5$ jeweils Wasserstoff, $R^4$ Isopropyl und $R^6$ Methyl bedeuten.

5. Optisches Aufzeichnungsmedium, enthaltend einen Träger sowie einen strahlungsempfindlichen, Farbstoff und gegebenenfalls Bindemittel enthaltenden dünnen Beschichtungsfilm, dadurch gekennzeichnet, daß der Farbstoff die Formel I

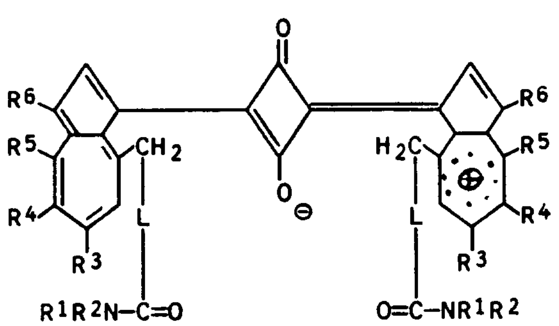

(I)

aufweist, in der

| | |
|---|---|
| L | $C_1$-$C_{12}$-Alkylen, das gegebenenfalls durch Phenyl substituiert ist, |

| | |
|---|---|
| $R^1$ und $R^2$ | gleich oder verschieden sind und unabhängig voneinander jeweils Wasserstoff, gegebenenfalls substituiertes $C_1$-$C_{20}$-Alkyl, $C_5$-$C_7$-Cycloalkyl, gegebenenfalls substituiertes Phenyl, 2,2,6,6-Tetramethylpiperidin-4-yl oder Cyclohexylaminocarbonyl und |
| $R^3$, $R^4$, $R^5$ und $R^6$ | gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, |

bedeuten, mit der Maßgabe, daß wenn $R^6$ Wasserstoff bedeutet, an einem oder beiden Azulenringen die Ringpositionen der Substituenten $CH_2$-L-CO-$NR^1R^2$ und $R^5$ innerhalb eines Azulenringes auch gegeneinander vertauscht sein können.

**6.** Optisches Aufzeichnungsmedium nach Anspruch 5, dadurch gekennzeichnet, daß $R^3$, $R^4$, $R^5$ und $R^6$ jeweils $C_1$-$C_6$-Alkyl oder Wasserstoff bedeuten.

**7.** Optisches Aufzeichnungsmedium nach Anspruch 5, dadurch gekennzeichnet, daß $R^3$ und $R^5$ jeweils Methyl und $R^4$ und $R^6$ jeweils Wasserstoff bedeuten.

**8.** Optisches Aufzeichnungsmedium nach Anspruch 5, dadurch gekennzeichnet, daß $R^3$ und $R^5$ jeweils Wasserstoff, $R^4$ Isopropyl und $R^6$ Methyl bedeuten.

**9.** Amidgruppen aufweisende Azulene der Formel II

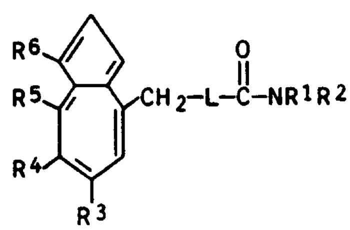

(II),

in der

| | |
|---|---|
| L | $C_1$-$C_{12}$-Alkylen, das gegebenenfalls durch Phenyl substituiert ist, |
| $R^1$ und $R^2$ | gleich oder verschieden sind und unabhängig voneinander jeweils Wasserstoff, gegebenenfalls substituiertes $C_1$-$C_{20}$-Alkyl, $C_5$-$C_7$-Cycloalkyl, gegebenenfalls substituiertes Phenyl, 2,2,6,6-Tetramethylpiperidin-4-yl oder Cyclohexylaminocarbonyl und |
| $R^3$, $R^4$, $R^5$ und $R^6$ | gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, |

bedeuten.

## Claims

**1.** An amido-containing azulenesquaric acid dye of the formula I

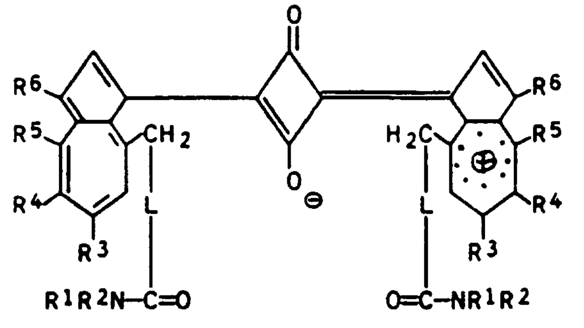

(I)

where

L is $C_1$-$C_{12}$-alkylene which may be substituted by phenyl,

$R^1$ and $R^2$ are identical or different and each is independently of the other hydrogen, substituted or unsubstituted $C_1$-$C_{20}$-alkyl, $C_5$-$C_7$-cycloalkyl, substituted or unsubstituted phenyl, 2,2,6,6-tetramethylpiperidin-4-yl or cyclohexylaminocarbonyl and

$R^3$, $R^4$, $R^5$ and $R^6$ are identical or different and each is independently of the others hydrogen or $C_1$-$C_{12}$-alkyl which may be substituted by halogen, $C_1$-$C_{12}$-alkoxy, phenyl, substituted phenyl, $C_1$-$C_{12}$-alkoxycarbonyl or by cyano,

with the proviso that when $R^6$ is hydrogen the positions of the substituents $CH_2$-L-CO-$NR^1R^2$ and $R^5$ on either or both azulene rings may also be interchanged with each other within an azulene ring.

2. An azulenesquaric acid dye as claimed in claim 1, wherein $R^3$, $R^4$, $R^5$ and $R^6$ are each $C_1$-$C_6$-alkyl or hydrogen.

3. An azulenesquaric acid dye as claimed in claim 1, wherein $R^3$ and $R^5$ are each methyl and $R^4$ and $R^6$ are each hydrogen.

4. An azulenesquaric acid dye as claimed in claim 1, wherein $R^3$ and $R^5$ are each hydrogen, $R^4$ is isopropyl and $R^6$ is methyl.

5. An optical recording medium comprising a base material and a radiation-sensitive thin coating film which contains a dye with or without a binder, wherein the dye has the formula I

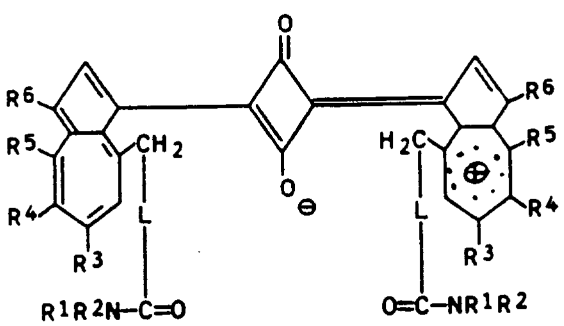

(I)

where

L is $C_1$-$C_{12}$-alkylene which may be substituted by phenyl,

$R^1$ and $R^2$ are identical or different and each is independently of the other hydrogen, substituted or unsubstituted $C_1$-$C_{20}$-alkyl, $C_5$-$C_7$-cycloalkyl, substituted or unsubstituted phenyl, 2,2,6,6-tetramethylpiperidin-4-yl or cyclohexylaminocarbonyl and

$R^3$, $R^4$, $R^5$ and $R^6$ are identical or different and each is independently of the others hydrogen or $C_1$-$C_{12}$-alkyl which may be substituted by halogen, $C_1$-$C_{12}$-alkoxy, phenyl, substituted phenyl, $C_1$-$C_{12}$-alkoxycarbonyl or by cyano,

with the proviso that when $R^6$ is hydrogen the positions of the substituents $CH_2$-L-CO-$NR^1R^2$ and $R^5$ on either or both azulene rings may also be interchanged with each other within an azulene ring.

6. An optical recording medium as claimed in claim 5, wherein $R^3$, $R^4$, $R^5$ and $R^6$ are each $C_1$-$C_6$-alkyl or hydrogen.

7. An optical recording medium as claimed in claim 5, wherein $R^3$ and $R^5$ are each methyl and $R^4$ and $R^6$ are each hydrogen.

8. An optical recording medium as claimed in claim 5, wherein $R^3$ and $R^5$ are each hydrogen, $R^4$ is isopropyl and $R^6$ is methyl.

**9.** An amido-containing azulene of the formula II

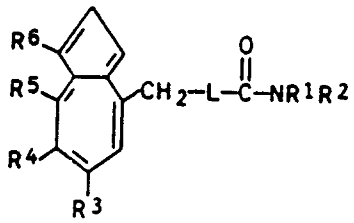

(II)

where

L is $C_1$-$C_{12}$-alkylene which may be substituted by phenyl,

$R^1$ and $R^2$ are identical or different and each is independently of the other hydrogen, substituted or unsubstituted $C_1$-$C_{20}$-alkyl, $C_5$-$C_7$-cycloalkyl, substituted or unsubstituted phenyl, 2,2,6,6-tetramethylpiperidin-4-yl or cycloherylaminocarbonyl and

$R^3$, $R^4$, $R^5$ and $R^6$ are identical or different and each is independently of the others hydrogen or $C_1$-$C_{12}$-alkyl which may be substituted by halogen, $C_1$-$C_{12}$-alkoxy, phenyl, substituted phenyl, $C_1$-$C_{12}$-alkoxycarbonyl or by cyano.

## Revendications

**1.** Colorants d'acide azulènequadratique comportant des groupements amide, de formule I

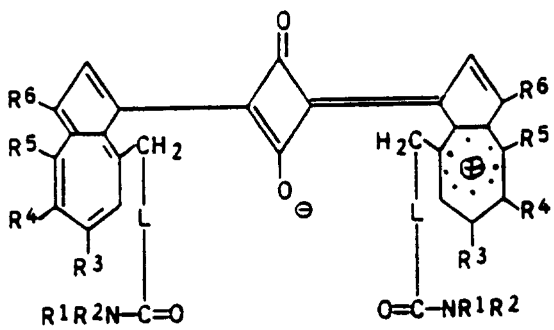

(I),

dans laquelle

L représente un reste alkylène en $C_1$-$C_{12}$ qui est éventuellement substitue par un radical phényle,

$R^1$ et $R^2$ sont identiques ou différents et représentent chacun, independamment l'un de l'autre, un atome d'hydrogène, un reste alkyle en $C_1$-$C_{20}$ éventuellement substitué, cycloalkyle en $C_5$-$C_7$, phényle éventuellement substitue, 2,2,6,6-tétraméthylpipéridine-4-yle ou cyclohexylaminocarbonyle et

$R^3$, $R^4$, $R^5$ et $R^6$ sont identiques ou différents et représentent chacun, indépendamment les uns des autres, un atome d'hydrogène ou un reste alkyle en $C_1$-$C_{12}$ qui est éventuellement substitué par un atome d'halogène ou par un groupement alcoxy en $C_1$-$C_{12}$, phényle, phényle substitué, (alcoxy en $C_1$-$C_{12}$)carbonyle ou cyano,

étant spécifié que quand $R^6$ représente un atome d'hydrogène, les positions des substituants $CH_2$-L-CO-$NR^1R^2$ et $R^5$ sur un noyau azulène peuvent aussi être interverties sur l'un ou sur l'un et l'autre des noyaux azulène.

**2.** Colorants d'acide azulènequadratique selon la revendication 1, caractérisés en ce que $R^3$, $R^4$, $R^5$ et $R^6$ représentent chacun un reste alkyle en $C_1$-$C_6$ ou un atome d'hydrogène.

3. Colorants d'acide azulènequadratique selon la revendication 1, caractérisés en ce que $R^3$ et $R^5$ représentent chacun un reste méthyle et $R^4$ et $R^6$ représentent chacun un atome d'hydrogène.

4. Colorants d'acide azulènequadratique selon la revendication 1, caractérisés en ce que $R^3$ et $R^5$ représentent chacun un atome d'hydrogène, $R^4$ représente un reste isopropyle et $R^6$ représente un reste méthyle.

5. Support d'enregistrement optique, comprenant un substrat et un film mince de revêtement sensible au rayonnement qui contient un colorant et éventuellement un liant, caractérisé en ce que le colorant répond à la formule I

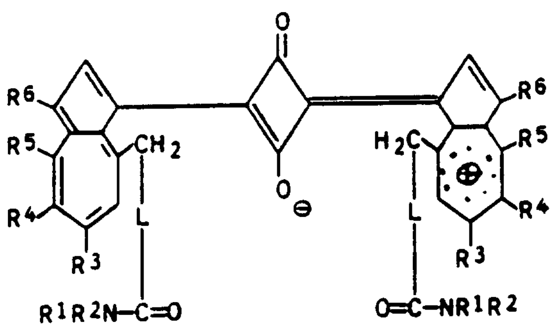

(I)

dans laquelle

| | |
|---|---|
| L | représente un reste alkylène en $C_1$-$C_{12}$ qui est éventuellement substitué par un radical phényle, |
| $R^1$ et $R^2$ | sont identiques ou différents et représentent chacun, indépendamment l'un de l'autre, un atome d'hydrogène, un reste alkyle en $C_1$-$C_{20}$ éventuellement substitué, cycloalkyle en $C_5$-$C_7$, phényle éventuellement substitué, 2,2,6,6-tétraméthylpipéridine-4-yle ou cyclohexylaminocarbonyle et |
| $R^3$, $R^4$, $R^5$ et $R^6$ | sont identiques ou différents et représentent chacun, indépendamment les uns des autres, un atome d'hydrogène ou un reste alkyle en $C_1$-$C_{12}$ qui est éventuellement substitué par un atome d'halogène ou par un groupement alcoxy en $C_1$-$C_{12}$, phényle, phényle substitué, (alcoxy en $C_1$-$C_{12}$)carbonyle ou cyano, |

étant spécifié que quand $R^6$ représente un atome d'hydrogène, les positions des substituants $CH_2$-L-CO-$NR^1R^2$ et $R^5$ sur un noyau azulène peuvent aussi être interverties sur l'un ou sur l'un et l'autre des noyaux azulène.

6. Support d'enregistrement optique selon la revendication 5, caractérisé en ce que $R^3$, $R^4$, $R^5$ et $R^6$ représentent chacun un reste alkyle en $C_1$-$C_6$ ou un atome d'hydrogène.

7. Support d'enregistrement optique selon la revendication 5, caractérisé en ce que $R^3$ et $R^5$ représentent chacun un reste méthyle et $R^4$ et $R^6$ représentent chacun un atome d'hydrogène.

8. Support d'enregistrement optique selon la revendication 5, caractérise en ce que $R^3$ et $R^5$ représentent chacun un atome d'hydrogène, $R^4$ représente un reste isopropyle et $R^6$ représente un reste méthyle.

**9.** Azulènes comportant des groupements amide, de formule II

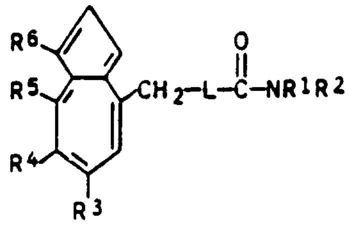

(II),

dans laquelle

L représente un reste alkylène en $C_1$-$C_{12}$ qui est éventuellement substitué par un radical phényle,

$R^1$ et $R^2$ sont identiques ou différents et représentent chacun, indépendamment l'un de l'autre, un atome d'hydrogène, un reste alkyle en $C_1$-$C_{20}$ éventuellement substitué, cycloalkyle en $C_5$-$C_7$, phényle éventuellement substitué, 2,2,6,6-tétraméthylpipéridine-4-yle ou cyclohexylaminocarbonyle et

$R^3$, $R^4$, $R^5$ et $R^6$ sont identiques ou différents et représentent chacun, indépendamment les uns des autres, un atome d'hydrogène ou un reste alkyle en $C_1$-$C_{12}$ qui est éventuellement substitué par un atome d'halogéne ou par un groupement alcoxy en $C_1$-$C_{12}$, phényle, phényle substitué, (alcoxy en $C_1$-$C_{12}$)carbonyle ou cyano.